# FASCICULE DE BREVET EUROPEEN

(11) **EP 2 942 626 B1**
(45) Date de publication et mention de la délivrance du brevet: **31.10.2018**
(21) Numéro de dépôt: 15168326.5
(22) Date de dépôt: 02.07.2008
(51) Int. Cl.: G01N 33/543, G01N 33/545

(54) **DISPOSITIF ET PROCEDE D'IDENTIFICATION ET DE DETERMINATION DE GROUPES SANGUINS**
VORRICHTUNG UND VERFAHREN ZUR IDENTIFIKATION UND BESTIMMUNG VON BLUTGRUPPEN
DEVICE AND METHOD FOR IDENTIFYING AND DETERMINING BLOOD GROUPS

(30) Priorité: 02.07.2007 FR 0704741
(43) Date de publication de la demande: 11.11.2015
(62) Demande divisionnaire de: 08806156.9
(73) Titulaire: Diagast, 59120 Loos (FR)
(72) Inventeur: CHAIBI, Najim, 33140 Villenave d'Ornon (FR)
(74) Mandataire: Regimbeau

(56) Documents cités:
- WO-A-2004/103939
- WO-A1-2006/098803
- GB-A- 2 250 342
- US-A- 5 126 276
- US-A- 5 710 049
- US-A1- 2005 124 077
- KNIGHT R C ET AL: "Detection of red cell antibodies: Current and future techniques", BRITISH JOURNAL OF BIOMEDICAL SCIENCE, ROYAL SOCIETY OF MEDICINE SERVICES, LONDON, GB, vol. 52, no. 4, 1995, pages 297-305, XP008007278, ISSN: 0967-4845

## Description

La présente invention est relative à un dispositif pour l'identification et la détermination d'antigènes érythrocytaires de groupes sanguins et des anticorps plasmatiques correspondants à partir d'un échantillon de sang total.

L'invention vise également l'utilisation de ce dispositif, un procédé mettant en oeuvre ce dispositif, ainsi qu'un kit de détermination des groupes sanguins.

Le sang est un tissu conjonctif liquide présent chez l'homme et la plupart des animaux évolués. Malgré une composition cellulaire identique, il existe une variabilité des divers éléments du sang, définis par différents systèmes antigéniques appelés groupes sanguins.

En pratique on s'intéresse plus particulièrement aux groupes sanguins érythrocytaires, systèmes d'antigènes situés à la surface des globules rouges, tels que par exemple les systèmes ABO, Rhésus, Kell, Duffy, MNS, Lewis, etc.

Classiquement, la détermination d'un groupe sanguin se fait sur la reconnaissance antigène-anticorps. Lorsqu'un anticorps spécifique de l'antigène reconnaît celui-ci, il se fixe. Généralement les anticorps utilisés dans la reconnaissance de groupes sanguins sont des immunoglobulines IgM agglutinant les globules rouges.

Les techniques habituellement utilisées, consistent à rechercher et identifier la présence ou l'absence d'antigènes de groupe sanguin à la surface des érythrocytes ou à rechercher et identifier la présence ou l'absence d'anticorps anti-antigène de groupe sanguin dans le plasma.

En particulier, pour le système ABO, l'épreuve de Beth-vincent permet de déterminer les antigènes portés par les globules rouges, et l'épreuve complémentaire de Simonin-Michon ou contre épreuve-sérique permet de déterminer les anticorps circulant dans le sérum.

Dans l'épreuve de Beth-Vincent, les globules rouges de l'individu, obtenus après séparation de phase des cellules et du plasma, soit par centrifugation soit par décantation, sont mis en présence de réactifs d'anticorps de spécificité connue. Généralement ce test est rendu visible par observation d'agglutination des globules rouges lorsque les anticorps reconnaissent les antigènes érythrocytaires correspondants.

Dans l'épreuve de Simonin, le plasma de l'individu est mis en présence de globules rouges tests appartenant chacun à un groupe antigénique précis du système ABO. Il s'agit d'un test d'agglutination du plasma de l'individu avec des hématies tests.

Pour la recherche d'anticorps dits irréguliers ou RAI, il s'agit de détecter la présence ou l'absence dans le sang d'un individu d'immunoglobulines dirigées contre divers antigènes érythrocytaires de l'individu. Pour cela on cherche à mettre en évidence la fixation de ces immunoglobulines sur des globules rouges tests dont les antigènes sont connus, avec la technique de Coombs direct et indirect, la comparaison des résultats permettant de déduire la présence ou l'absence d'immunoglobulines.

Il existe un grand nombre de procédés et de dispositifs utilisés pour le phénotypage dans le domaine de l'immunologie-hématologie, les techniques pouvant être manuelles, sur plaque d'opaline, en tube ou en cupule de microplaque, ou encore complètement automatisées à l'aide de robot distributeur d'échantillon et de réactif, agitateur, incubateur et lecteur automatique. On connaît notamment deux techniques de référence : les techniques en microplaques et les techniques en filtration par gel-test.

Toutefois ces techniques existantes de phénotypage de groupes sanguins présentent de nombreux inconvénients.

Les techniques en microplaque par exemple nécessitent une phase d'agitation qui est critique car les multiples réactions simultanément présentes sur le support n'ont pas la même cinétique de remise en suspension. Elles doivent être réalisées sous contrôle visuel et il faut être particulièrement attentif aux phénomènes d'adhérence de certains réactifs.

De même, lors de la mise en oeuvre de techniques en filtration par gel-test, il existe également un risque de non détection de certaines agglutinations notamment lors de l'épreuve plasmatique du groupe ABO. Un autre inconvénient est la détection trop fréquente d'auto-anticorps en relation avec les préparations d'hématies-tests, en particulier celles traitées par des enzymes protéolytiques.

En outre, toutes ces techniques présentent un inconvénient majeur du fait qu'elles nécessitent une centrifugation préalable du sang total afin de séparer les éléments constitutifs du sang, étape contraignante qui augmente fortement le temps et le coût d'analyse, et qui nécessite l'utilisation de centrifugeuses encombrantes et difficiles à manipuler.

Afin de supprimer cette étape lourde de centrifugation, des variantes ont été développées, basées sur l'utilisation de particules magnétiques.

On peut citer à titre d'exemple la demande de brevet EP-0.351.857 qui décrit un procédé de dosage immunologique utilisant des marqueurs magnétisés tels que des anticorps ou des antigènes fixés sur des billes de latex magnétiques. Il est notamment décrit une technique RAI par immunoadhérence dans laquelle on applique un champ magnétique sur des hématies préalablement fixées au fond d'une cupule de microplaque, sensibilisées avec le sérum à tester, lavées et mélangées à des billes de latex magnétiques revêtues d'une anti-immunoglobuline.

On connaît également la demande EP-0.230.768 qui décrit un procédé de co-agrégation de particules magnétiques capables de se lier à une substance contenue dans un échantillon au moyen de composés polycationiques en présence d'un champ magnétique.

La demande britannique GB2250342 décrit un dispositif de détermination des groupes sanguins comportant des zones réactives qui ne comportent pas de membrane absorbante. Ce dispositif permet une détermination des groupes sanguins par un flux horizontal de l'échantillon testé.

Toutefois ces différentes techniques présentent également de nombreux inconvénients. Elles sont difficiles et longues à mettre en oeuvre, peu économiques et nécessitent l'utilisation de dispositifs complexes et peu mobiles ou ne permettent pas de tester différents échantillons simultanément..

Aussi, la présente invention vise à pallier les inconvénients de l'art antérieur en proposant un moyen simple et efficace pour le phénotypage de groupes sanguins érythrocytaires, et l'identification et la détermination des anticorps plasmatiques correspondants, présentant une précision et une exactitude au moins comparables à celles obtenues par les méthodes de référence.

En particulier l'objectif de l'invention est de proposer un système économique, facile d'utilisation, automatisable et mobile, capable de déterminer et de détecter rapidement des groupes sanguins érythrocytaires par immobilisation des globules rouges directement à partir d'un échantillon de sang total, sans utilisation d'un appareillage de centrifugation et/ou d'un appareil de mesure quelconque.

Pour répondre à cet objectif, la présente invention propose un dispositif pour l'identification et la détermination de groupes sanguins à partir d'un échantillon de sang total, comprenant un support solide qui comporte au moins une zone réactive, ladite zone réactive étant constituée par au moins une membrane poreuse polymère, présentant des pores de diamètre compris entre 1 et 20 µm, destinée à être imprégnée d'au moins un réactif de complexation qui est un réactif anticorps monoclonaux spécifiques des antigenes érythrocytaires de groupe sanguin ou des réactifs anti globulines permettant l'immobilisation des anticorps plasmatiques reconnaissant les érythrocytes, et une membrane absorbante, destinée à récupérer l'excès de sang de l'échantillon analysé. Le dispositif permet ainsi d'identifier et de déterminer des antigènes érythrocytaires de groupes sanguins et/ou des anticorps plasmatiques correspondants.

L'invention vise également l'utilisation de ce dispositif, ainsi qu'un procédé de phénotypage de groupes sanguins érythrocytaires par réaction d'immobilisation des globules rouges, mettant en oeuvre ce dispositif.

Avantageusement la présente invention permet de détecter et/ou de déterminer un groupe sanguin érythrocytaire rapidement, directement à partir de sang total, par un résultat de lecture par positivité, sans utiliser de centrifugeuse ou d'appareillage de mesure. Le dispositif permet une détection simultanée des antigènes de groupes sanguins et des anticorps plasmatiques correspondants.

Selon un dernier aspect, l'invention est également relative à un kit pour l'identification et la détermination d'antigènes érythrocytaires de groupes sanguins et des anticorps plasmatiques correspondants.

D'autres caractéristiques et avantages ressortiront de la description qui va suivre de l'invention, description donnée à titre d'exemple uniquement, en regard des dessins annexés sur lesquels :
- la figure 1 représente un schéma du dispositif selon l'invention vu en perspective,
- la figure 2a représente une coupe schématique d'un premier mode de réalisation d'une zone réactive d'un dispositif selon l'invention,
- la figure 2b représente une coupe schématique d'un second mode de réalisation d'un dispositif selon l'invention, et
- figure 3 représente une vue en perspective d'un mode de réalisation du dispositif selon l'invention.

Le dispositif selon l'invention est destiné à la détermination et/ou la détection de groupes sanguins érythrocytaires à partir d'un échantillon de sang total.

Comme représenté sur les figures, il comprend un support solide 1 comportant au moins une zone réactive 4, ladite zone réactive étant constituée par au moins :
- une membrane poreuse 3 polymère, présentant des pores de diamètre compris entre 1 et 20 µm, destinée à être imprégnée d'au moins un réactif de complexation qui est un réactif anticorps monoclonaux spécifiques des antigenes érythrocytaires de groupe sanguin
   ou des réactifs anti globulines permettant l'immobilisation des anticorps plasmatiques reconnaissant les érythrocytes , et
- une membrane absorbante 2, destinée à récupérer l'excès de sang de l'échantillon analysé.

La membrane poreuse polymère 3 présente des pores de diamètre compris entre 1 et 20 µm, préférentiellement entre 1 et 14 µm.

Selon un mode de réalisation préféré la membrane poreuse 3 est transparente. Il peut s'agir d'une membrane en polyéthylène haute densité, qui présente des caractéristiques particulièrement adaptées à l'invention en particulier pour l'activation et l'immobilisation des anticorps. Encore plus préférentiellement, la membrane poreuse 3 est telle qu'elle présente une bonne résistance, une vitesse de migration rapide correspondant à l'adsorption d'une goutte de sang total en moins de 10 secondes, et une porosité moyenne de 7µm.

La membrane 3 est destinée à être imprégnée de réactifs de complexation qui sont un réactif anticorps monoclonaux spécifiques des des antigenes érythrocytaires de groupe sanguin ou des réactifs anti globulines permettant l'immobilisation des anticorps plasmatiques reconnaissant les érythrocytes. A titre d'exemple, dans le cas où le dispositif est utilisé pour une épreuve de Beth-Vincent, la membrane 3 est imprégnée de réactifs anticorps monoclonaux, tels que des anticorps anti-Al, anti-A2, anti-B, anti-AB ou anti-D ; dans le cas où le dispositif est utilisé pour une épreuve de Simonin, la membrane 3 est imprégnée de réactifs antiglobulines ou de lectines. Les réactifs de complexation sont liés de manière spécifique sur la zone d'activité.

Les réactifs de complexation sont fixés sur la membrane 3 par tout moyen approprié. Notamment ils peuvent être fixés par adsorption passive et sélective. Cette sélectivité améliore la justesse et la précision du test.

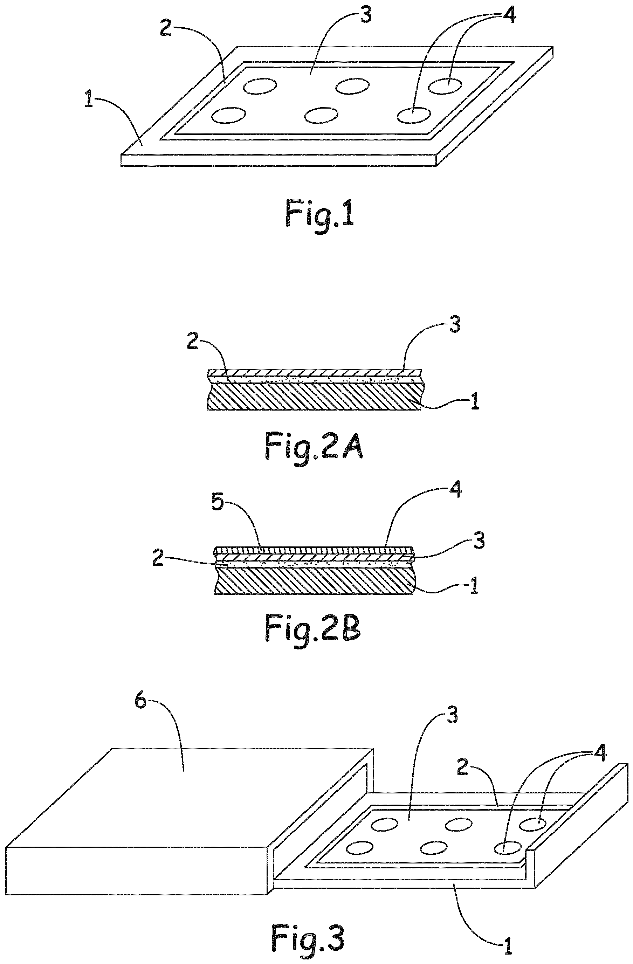

Selon une variante illustrée sur la figure 2b, la ou les zones réactives 4 du dispositif comprennent également un filtre 5. Ce filtre peut par exemple être constitué de fibres de verre de diamètre compris entre 1 et 7µm.

Lorsque le dispositif est utilisé pour l'identification et la détermination d'anticorps dans le plasma de l'individu, ce filtre 5 est préférentiellement imprégné par des réactifs capables d'agglutiner les érythrocytes, de façon à retenir les globules rouges et à ne laisser passer que le plasma lorsque du sang total est déposé. A titre d'exemple, ces réactifs peuvent être des agglutines de soja, lectines ou tout autre substance biologique ou non permettant d'agglutiner des globules rouges.

Ces réactifs d'agglutination sont fixés sur le filtre 5 par tout moyen approprié. Notamment ils peuvent être fixés par adsorption.

Selon un mode de réalisation particulier de l'invention représenté sur la figure 3, le dispositif comprend également un réservoir 6 contenant une solution de lavage et/ou de révélation. Ce réservoir peut être fixé de manière solidaire au dispositif.

Le dispositif selon l'invention peut être utilisé pour déterminer et/ou détecter des groupes sanguins, en particulier des groupes sanguins ABO, Rhésus et RAI, à partir d'un échantillon de sang total.

Le dispositif selon l'invention peut être utilisé en particulier pour la mise en oeuvre d'un procédé de phénotypage de groupes sanguins érythrocytaires par réaction d'immobilisation d'érythrocytes, comprenant les étapes suivantes :
- déposer un échantillon de sang total sur une zone réactive 4,
- laisser réagir au moins 30 secondes, et
- appliquer une solution de lavage afin d'éluer les éléments non liés à la membrane poreuse 3.

Quand le sang est déposé sur la zone réactive 4, les éléments du sang capables de se lier spécifiquement aux réactifs de complexation de la membrane poreuse 3 sont captés sur ladite membrane. Lorsque la solution de lavage est appliquée, telle qu'une solution de lavage PBS-tween, les éléments du sang non fixés sur la membrane 3 sont éliminés vers la membrane tampon 2.

La lecture du résultat se fait par positivité de manière macroscopique. Si des hématies sont immobilisées sur la membrane poreuse 3, le résultat se traduit par une coloration de la zone réactive 4.

Selon un mode de réalisation de l'invention particulièrement adapté au phénotypage de groupes sanguins ABO, Rhésus et RAI, la membrane 3 est imprégnée par des réactifs anticorps anti-antigènes spécifiques des érythrocytes, tels que par exemple que des anticorps anti-Al, anti-A2, anti-B, anti-AB ou anti-D. La membrane 3 joue alors le rôle d'un piège pour les globules rouges permettant leur fixation sur les immunoglobulines fixées au préalable sur la zone réactive. Les réactifs anticorps liés de manière spécifique sur la zone réactive permettent une captation des globules rouges qui entraîne une immobilisation de ces derniers. En outre, ce phénomène est amplifié par une agglutination de fait, due aux quelques résidus d'anticorps non élués par la solution de lavage.

Lorsque les globules rouges de l'échantillon sanguin déposé sur la zone réactive portent l'antigènes correspondant à l'anticorps présent dans la zone réactive, il y a une réaction de captation et d'immobilisation des globules rouges qui se traduit visuellement par une coloration rouge de la zone réactive.

Lorsque les globules rouges ne sont pas reconnus par les anticorps correspondants, ils sont élués vers la membrane tampon 2 et il y a une absence de coloration sur la zone réactive.

Avantageusement ce procédé n'utilise pas de technique de coloration pour la révélation et la lecture du résultat, ni de dispositif particulier pour observer une agglutination.

En outre, le procédé selon l'invention permet d'éviter des pseudos agglutinations source d'erreurs notamment lors des pratiques en gel-test de l'art antérieur.

Selon un autre mode de réalisation de l'invention particulièrement adapté au phénotypage de groupes ABO, Rhésus et RAI, la membrane 3 est imprégnée par des réactifs antiglobulines permettant l'immobilisation de tous les anticorps plasmatiques de l'individu.

Le sang total est déposé sur une zone réactive 4 munie d'un filtre 5 imprégné par des réactifs d'agglutination. Les hématies s'agglutinent au niveau de ce filtre et seul le plasma atteint la membrane poreuse 3.

La membrane 3 joue alors le rôle d'un piège pour les anticorps plasmatiques circulants qui se lient aux réactifs antiglobulines fixés au préalable.

On dépose ensuite des hématies tests d'antigénicité sur la membrane 3, on laisse agir au moins 30 secondes et on applique une solution de lavage afin d'éluer les éléments non liés à la membrane poreuse 3.

Lorsque les anticorps plasmatiques de l'échantillon sanguin liés à la membrane 3 par fixation aux antiglobulines, reconnaissent les hématies-tests correspondant, ces dernières se fixent et s'immobilisent au niveau de la membrane poreuse 3. Le résultat se traduit visuellement par une coloration liée à l'immobilisation des hématies tests appliquées.

Lorsque les anticorps plasmatiques immobilisés dans le support ne reconnaissent pas les hématies-tests, ces dernières sont éliminées par la solution de lavage vers la membrane tampon. Préférentiellement, afin d'augmenter la sensibilité du test, les solutions d'hématies peuvent être reconcentrées.

Avantageusement le dispositif et le procédé selon l'invention sont fiables, faciles d'utilisation et permettent un phénotypage de groupe sanguin directement à partir d'un échantillon de sang total sans préparation préalable. Le dispositif est aisément transportable et l'utilisateur peut mettre en oeuvre le procédé dans n'importe quel lieu, sans appareillage de mesure ni appareillage de centrifugation.

Les exemples qui suivent montrent un mode de réalisation possible du dispositif selon l'invention ainsi que son utilisation pour deux épreuves complémentaires de détermination de groupe sanguin : une épreuve globulaire et pour une épreuve sérique.

### Exemple 1 : Mode de réalisation d'un dispositif selon l'invention

### Membrane poreuse

La membrane poreuse 3 est une membrane polyéthylène haute densité présentant un volume des pores de 40 à 45%, un diamètre de pores moyen de 7µm, une porosité distribuée entre 1 et 14 µm.

Elle est délimitée sous forme de disques de 6mm de diamètre et de 1mm d'épaisseur.

Les disques sont lavés avec de l'éthanol pur afin d'enlever toutes les impuretés de bas poids moléculaire et sont mis à sécher dans une étuve à 60°C.

### Filtre

La membrane filtre 5 est constituée notamment de fibres de verre de diamètre de 1 à 7 µm.

Elle est délimitée sous forme de disques de 6mm de diamètre

### Membrane absorbante

La membrane absorbante 2 sont des membranes obtenues chez Whatman avec une capacité s'absorption de 198 mg/cm2.

Elle est délimitée sous forme de disques de 6mm de diamètre et un volume d'absorption équivalent à 50 µl de sang total.

### Support solide

Le support solide 1 permettant d'accueillir les différentes membranes, est un support en plastique de 12cm de longueur et de 8 cm de largeur.

Il est destiné à contenir 72 zones réactives disposées en 8 colonnes, avec chaque zone comprenant un filtre 5, une membrane 3 et une membrane 2. Les colonnes sont destinées à l'identification et la détermination :
- des antigènes Al pour la colonne 1,
- des antigènes B pour la colonne 2,
- des antigènes AB pour la colonne 3,
- des antigènes D pour la colonne 5,
- des anticorps plasmatiques anti-A pour la colonne 6,
- des anticorps plasmatiques anti-B pour la colonne 7.

Les colonnes 4 et 8 sont des contrôles positifs.

### Réactifs anticorps

Les réactifs anticorps anti-Al, anti-B, anti-AB et anti-D sont des réactifs anticorps monoclonaux purifiés.

Ils sont contenus dans une solution tampon PBS.

Ils sont ensuite déposés sur les disques de membrane 3 destinés à être disposés au niveau de la colonne 1 pour anti-Al, de la colonne 2 pour anti-B, de la colonne 3 pour anti-AB et de la colonne 5 pour anti-D, puis sont mis à sécher.

Un solution de lavage contenant du PBS tween est appliquée sur chaque zone réactive afin d'éliminer les anticorps non lier à la membrane 3.

### Réactifs antiglobulines

Les réactifs antiglobulines sont des réactifs anticorps anti-fc purifiés.

Les réactifs antiglobulines sont ensuite déposés sur les disques de membrane 3 destinés à être disposés au niveau de la colonne 6 et de la colonne 7, puis sont mis à sécher.

Un solution de lavage contenant du PBS tween est appliquée sur chaque zone réactive afin d'éliminer les antiglobulines non liées spécifiquement à la membrane 3.

### Réactifs agglutinant les globules rouges

Des agglutines de soja sont préparées à raison de 90mg/L dans du tampon TRIS à pH 7.

Elles sont ensuite déposées et imprégnées sur le filtre 5 uniquement au niveau de la colonne 6 et de la colonne 7, puis sont mis à sécher.

### Exemple 2 : Mise en oeuvre du procédé pour les colonnes 1 à 5

Une goutte de sang total est déposée sur chaque zone réactive.

On attend entre 30 secondes et une minute, puis on applique une solution de lavage pour éluer les éléments du sang non liés spécifiquement.

Lorsque l'échantillon est déposé sur les zones réactives des colonnes 1 à 5, tous les éléments du sang traversent le filtre 5 pour arriver au niveau de la membrane poreuse 3, car la membrane 5 est dépourvue de réactifs d'agglutination des globules rouges. Au niveau de la membrane poreuse 3 si les globules rouges de l'échantillon sanguin portent l'antigène correspondant à l'anticorps présent dans la zone réactive, il y a une réaction de captation et d'immobilisation des globules rouges qui se traduit par une coloration rouge de la zone réactive. Lorsque les globules rouges ne sont pas reconnus ils sont élués vers la membrane tampon 2, ce qui se traduit visuellement par une absence de coloration de la zone réactive.

### Exemple 3 : Mise en oeuvre du procédé pour les colonnes 6 à 8

Une goutte de sang total est déposée sur chaque zone réactive des colonnes 6 à 8.

Les globules rouges s'agglutinent au niveau du filtre 5 et une certaine quantité de plasma traverse le filtre et arrive au niveau de la membrane poreuse 3 imprégnée d'antiglobulines.

Des hématies tests d'antigénicités connus sont ensuite déposées sur chaque zone réactive.

On attend entre 30 secondes et une minute, puis on applique une solution de lavage pour éluer les éléments du sang non liés spécifiquement.

Lorsque les anticorps plasmatiques de l'échantillon sanguin reconnaissent les hématies-tests correspondantes, ces dernières se fixent au niveau de la zone réactive. Le résultat se traduit par une coloration de la zone réactive.

Lorsque les anticorps plasmatiques immobilisés ne reconnaissent pas les hématies-tests, ces dernières sont éliminées par la solution de lavage vers la membrane tampon 2.

Selon un autre mode de réalisation les hématies tests sont incorporées à la membrane poreuse 3 et maintenues humide par un dispositif de protection contre la déshydratation. Lorsque l'on applique du sang total au niveau d'une zone réactive, les anticorps plasmatiques circulant qui passent à travers le filtre 5 s'immobilisent sur les antigènes membranaires correspondants au niveau de la membrane 3 ou sont élués par la solution de lavage.

Avantageusement, l'invention permet de réaliser un test de Beth-Vincent et un test de Simonin sur un même dispositif sans appareillage spécifique et sans modification préalable de l'échantillon de sang.

Selon un dernier aspect, l'invention est également relative à un kit pour l'identification et la détermination d'antigènes érythrocytaires de groupes sanguins et des anticorps plasmatiques correspondants.

Ce kit comprend au moins un dispositif tel que décrit précédemment, des réactifs anticorps permettant la détermination et l'identification des antigènes érythrocytaires des groupes sanguins, et des hématies tests permettant la détermination et l'identification des anticorps plasmatiques circulants.

## Revendications

1. Dispositif pour l'identification et la détermination de groupes sanguins à partir d'un échantillon de sang total, **caractérisé en ce qu'**il comprend un support solide (1) comportant au moins une zone réactive (4), ladite zone réactive étant constituée par au moins :
- une membrane poreuse (3) polymère, présentant des pores de diamètre compris entre 1 et 20 µm, imprégnée d'un réactif anticorps monoclonaux spécifiques des antigènes érythrocytaires ou des réactifs anti globulines permettant l'immobilisation des anticorps plasmatiques reconnaissant les érythrocytes, et
- une membrane absorbante (2), destinée à récupérer l'excès de sang de l'échantillon analysé.

2. Dispositif selon la revendication 1, **caractérisé en ce que** la membrane poreuse (3) présente des pores de diamètre compris entre 1 et 14 µm.

3. Dispositif selon la revendication 1 ou 2, **caractérisé en ce que** la membrane poreuse (3) est une membrane transparente.

4. Dispositif selon l'une des revendications 1 à 3, **caractérisé en ce que** la membrane poreuse (3) présente une bonne résistance, une vitesse de migration rapide correspondant à l'adsorption d'une goutte de sang total en moins de 10 secondes et une porosité moyenne de 7 µm.

5. Dispositif selon l'une des revendications 1 à 4, **caractérisé en ce que** la membrane poreuse (3) est une membrane en polyéthylène haute densité.

6. Dispositif selon l'une des revendications 1 à 5, **caractérisé en ce qu'**au moins une zone réactive (4) comprend également un filtre (5).

7. Dispositif selon la revendication 6, **caractérisé en ce que** le filtre (5) est constitué de fibres de verre de diamètre compris entre 1 et 7 µm.

8. Dispositif selon l'une des revendications 1 à 7, **caractérisé en ce que** la membrane poreuse (3) est imprégnée par des réactifs antiglobulines permettant l'immobilisation des anticorps plasmatiques circulants.

9. Dispositif selon l'une des revendications 1 à 8, **caractérisé en ce qu'**il comprend un réservoir (6) contenant une solution de lavage et/ou de révélation.

10. Utilisation du dispositif selon l'une des revendications 1 à 9, pour identifier et déterminer des groupes sanguins ABO, Rhésus et/ou RAI à partir d'un échantillon de sang total.

11. Procédé de phénotypage de groupes sanguins érythrocytaires, **caractérisé en ce qu'**il comprend les étapes suivantes :
- déposer un échantillon de sang total sur une zone réactive (4) d'un dispositif selon l'une des revendications 1 à 9,
- laisser réagir au moins 30 secondes, et
- appliquer une solution de lavage afin d'éluer les éléments non liés à la membrane poreuse (3).

12. Procédé de phénotypage de RAI, **caractérisé en ce qu'**il comprend les étapes suivantes :
- déposer un échantillon de sang total sur une zone réactive (4) d'un dispositif selon les revendications 1 à 7, dont la membrane poreuse (3) a été imprégnée par des réactifs antiglobulines permettant l'immobilisation des anticorps plasmatiques circulants,
- déposer des hématies-tests d'antigénicité connue sur ladite zone réactive (4),
- laisser agir au moins 30 secondes, et
- appliquer une solution de lavage afin d'éluer les éléments non liés à la membrane poreuse (3).

13. Kit pour la détermination et/ou la détection des groupes sanguins ABO, Rhésus et/ou RAI à partir d'un échantillon de sang total, **caractérisé en ce qu'**il comprend au moins :
- un dispositif selon l'une des revendications 1 à 8,
- ,et
- des hématies tests permettant la détermination et l'identification des anticorps plasmatiques circulants.

## Patentansprüche

1. Vorrichtung zur Identifizierung und zur Bestimmung von Blutgruppen anhand einer Vollblutprobe, **dadurch gekennzeichnet, dass** sie einen festen Träger (1) umfasst, der mindestens eine reaktive Zone (4) umfasst, wobei die reaktive Zone besteht aus mindestens:
- einer porösen Polymermembran (3), die Poren eines Durchmessers im Bereich zwischen 1 und 20 µm aufweist, getränkt mit einem Reagenz monoklonaler Antikörper, die für die erythrozytären Antigene spezifisch sind, oder mit Antiglobulin-Reagenzien, die die Immobilisierung der die Erythrozyten erkennenden plasmatischen Antikörper ermöglichen, und
- einer absorbierenden Membran (2), die dazu vorgesehen ist, den Überschuss an Blut aus der analysierten Probe aufzufangen.

2. Vorrichtung nach Anspruch 1, **dadurch gekennzeichnet, dass** die poröse Membran (3) Poren eines Durchmessers im Bereich zwischen 1 und 14 µm aufweist.

3. Vorrichtung nach Anspruch 1 oder 2, **dadurch gekennzeichnet, dass** die poröse Membran (3) eine transparente Membran ist.

4. Vorrichtung nach einem der Ansprüche 1 bis 3, **dadurch gekennzeichnet, dass** die poröse Membran (3) eine gute Festigkeit, eine schnelle Migrationsgeschwindigkeit, die der Adsorption eines Tropfens Vollblut in weniger als 10 Sekunden entspricht, und eine mittlere Porosität von 7 µm aufweist.

5. Vorrichtung nach einem der Ansprüche 1 bis 4, **dadurch gekennzeichnet, dass** die poröse Membran (3) eine Membran aus hochdichtem Polyethylen ist.

6. Vorrichtung nach einem der Ansprüche 1 bis 5, **dadurch gekennzeichnet, dass** mindestens eine reaktive Zone (4) ebenfalls einen Filter (5) umfasst.

7. Vorrichtung nach Anspruch 6, **dadurch gekennzeichnet, dass** der Filter (5) aus Glasfasern eines Durchmessers im Bereich zwischen 1 und 7 µm besteht.

8. Vorrichtung nach einem der Ansprüche 1 bis 7, **dadurch gekennzeichnet, dass** die poröse Membran (3) mit Antiglobulin-Reagenzien getränkt ist, die die Immobilisierung der zirkulierenden plasmatischen Antikörper ermöglichen.

9. Vorrichtung nach einem der Ansprüche 1 bis 8, **dadurch gekennzeichnet, dass** sie ein Reservoir (6) umfasst, das eine Wasch- und/oder Entwicklungslösung enthält.

10. Verwendung der Vorrichtung nach einem der Ansprüche 1 bis 9, um AB0-, Rhesus- und/oder RAI-Blutgruppen anhand einer Vollblutprobe zu identifizieren und zu bestimmen.

11. Verfahren zur Phänotypisierung von erythrozytären Blutgruppen, **dadurch gekennzeichnet, dass** es die folgenden Schritte umfasst:
- Aufbringen einer Vollblutprobe auf eine reaktive Zone (4) einer Vorrichtung nach einem der Ansprüche 1 bis 9,
- Reagierenlassen für mindestens 30 Sekunden, und
- Anwenden einer Waschlösung, um die nicht an die poröse Membran (3) gebundenen Elemente zu eluieren.

12. Verfahren zur RAI-Phänotypisierung, **dadurch gekennzeichnet, dass** es die folgenden Schritte umfasst:
- Aufbringen einer Vollblutprobe auf eine reaktive Zone (4) einer Vorrichtung nach den Ansprüchen 1 bis 7, deren poröse Membran (3) mit Antiglobulin-Reagenzien getränkt wurde, die die Immobilisierung der zirkulierenden plasmatischen Antikörper ermöglichen,
- Aufbringen von Test-Erythrozyten von bekannter Antigenität auf die reaktive Zone (4),
- Reagierenlassen für mindestens 30 Sekunden, und
- Anwenden einer Waschlösung, um die nicht an die poröse Membran (3) gebundenen Elemente zu eluieren.

13. Set für die Bestimmung und/oder den Nachweis der AB0-, Rhesus- und/oder RAI-Blutgruppen anhand einer Vollblutprobe, **dadurch gekennzeichnet, dass** es mindestens umfasst:
- eine Vorrichtung nach einem der Ansprüche 1 bis 8,
- und
- Test-Erythrozyten, die das Bestimmen und das Identifizieren der zirkulierenden plasmatischen Antikörper ermöglichen.

## Claims

1. Device for identifying and determining blood groups using a whole blood sample, **characterised in that** it comprises a solid supporting member (1) comprising at least one reactive zone (4), said reactive zone consisting of at least:
- one polymer porous membrane (3) having pores between 1 and 20 µm in diameter, impregnated with monoclonal antibody reagents specifically directed against erythrocyte antigens or antiglobulin reagents suitable for immobilising plasma antibodies directed against erythrocytes, and
- an absorbent membrane (2), intended to retrieve the excess blood from the sample analysed.

2. Device according to claim 1, **characterised in that** the porous membrane (3) has pores between 1 and 14 µm in diameter.

3. Device according to claim 1 or 2, **characterised in that** the porous membrane (3) is a transparent membrane.

4. Device according to any one of claims 1 to 3, **characterized in that** the porous membrane (3) has a good resistance, a rapid rate of migration corresponding to the adsorption of a drop of whole blood in less than 10 seconds, and a mean porosity of 7 µm.

5. Device according to any one of claims 1 to 4, **characterized in that** the porous membrane (3) a high-density polyethylene membrane.

6. Device according to any one of claims 1 to 5, **characterized in that** at least one reactive zone (4) also comprises a filter (5).

7. Device according to claim 6, **characterised in that** the filter (5) consists of glass fibres between 1 and 7 µm in diameter.

8. Device according to any one of claim 1 to 7, **characterised in that** the porous membrane (3) is impregnated with antiglobulin reagents suitable for immobilising circulating plasma antibodies.

9. Device according to any of claims 1 à 8, **characterised in that** it comprises a container (6) containing a washing and/or detection solution.

10. Use of the device according to any of claims 1 to 9, for identifying and determining ABO, Rhesus blood groups and/or irregular antibodies (IAT) using a whole blood sample.

11. Method for phenotyping erythrocyte blood groups, **characterised in that** it comprises the following steps:
- depositing a whole blood sample onto a reactive zone (4) of a device according to any of claims 1 to 9,
- leaving to react for at least 30 seconds, and
- applying a washing solution so as to elute the elements not bound to the porous membrane (3).

12. Method for phenotyping irregular antibodies (IAT), **characterised in that** it comprises the following steps:
- depositing a whole blood sample onto a reactive zone (4) of a device according to claims 1 to 7, wherein the porous membrane (3) has been impregnated with antiglobulin reagents suitable for immobilising circulating plasma antibodies,
- depositing test blood cells of known antigenicity onto said reactive zone (4),
- leaving to stand for at least 30 seconds, and
- applying a washing solution so as to elute the elements not bound to the porous membrane (3).

13. Kit for determining and/or detecting ABO, Rhesus blood groups and/or irregular antibodies (IAT) using a whole blood sample, **characterised in that** it comprises at least:
- one device according to any of claims 1 to 8, and
- test blood cells suitable for determining and identifying circulating plasma antibodies.
